# EUROPÄISCHE PATENTSCHRIFT

(11) **EP 0 167 010 B1**
(45) Veröffentlichungstag und Bekanntmachung des Hinweises auf die Patenterteilung: **27.01.1993**
(21) Anmeldenummer: 85107004.5
(22) Anmeldetag: 05.06.1985
(51) Int. Cl.: G01N 29/00, G01N 33/34

(54) **Vorrichtung zur Bestimmung des Flächengewichts von blattförmigem Material**
Apparatus for measuring the surface weight of sheet material
Appareil pour mesurer le poids surfacique d'un matériau en forme de feuille

(30) Priorität: 04.07.1984 DE 3424652
(43) Veröffentlichungstag der Anmeldung: 08.01.1986
(73) Patentinhaber: GAO Gesellschaft für Automation und Organisation mbH, 81307 München (DE)
(72) Erfinder: Wunderer, Bernd, D-8000 München 40 (DE)
(74) Vertreter: Klunker . Schmitt-Nilson . Hirsch

(56) Entgegenhaltungen:
- EP-A- 0 098 115
- DE-A- 1 548 170
- DE-C- 882 150
- GB-A- 1 143 776
- US-A- 2 912 854

## Beschreibung

Die Erfindung betrifft eine Vorrichtung zur dynamischen Bestimmung des lokalen Flächengewichts von blattförmigem Material, beispielsweise Papier, bei der das blattförmige Material mit Schallwellen aus einem Schallsender beaufschlagt wird, der durch das blattförmige Material transmittierte und/oder reflektierte Schallanteil mit Hilfe eines Empfängers gemessen und aus dem Meßsignal das Flächengewicht bestimmt wird.

Aus der DE-A 15 48 170 ist ein Gerät zum Messen des Flächengewichts von blattförmigen Material bekannt. Das Meßgut wird mit Ultraschall bestrahlt und die transmittierte oder reflektierte Ultraschallenergie gemessen. Der Ultraschallsender arbeitet kontinuierlich oder im Impulsbetrieb. Zur Vermeidung von störenden Überlagerungen der Schallwellen, die zu stehenden Wellen und Echobildungen führen können, wird das den Sender und Empfänger aufnehmende Gehäuse mit einem schalldämpfenden Material ausgekleidet. Durch diese Maßnahme können zwar die Reflexionen an der Gehäusewand unterdrückt werden, nachteilig ist jedoch, daß es noch zu Ein- bzw. Mehrfachreflexionen an Sender, Empfänger und Meßgut kommen kann. Besonders bei der Dickenmessung von Papier können die Reflexionen am Meßgut zu erheblichen Verfälschungen des Meßwertes führen, da bei Papier nur ein sehr geringer Teil der Senderschallwelle durch das Papier transmittiert wird, während der weitaus größere Teil reflektiert wird und sich der Senderschallwelle überlagert.

Zur Vermeidung von Fehlern bei der Flächengewichtsbestimmung, die aufgrund von Reflexionen an Sender, Empfänger und Meßgut auftreten können, wird in der DE-OS 30 48 710 vorgeschlagen, den Sender impulsförmig anzusteuern und den Empfänger nur während eines kurzen Zeitfensters zu aktivieren. Das Zeitfenster wird derart gesetzt, daß der Empfänger erst eingeschaltet wird, wenn der Primärschall des Senders ankommt und ausgeschaltet wird, sobald die ersten systembedingten Störungen (Reflexionen) auftreten. Der Sender, der durch einen kurzen elektrischen Impuls angesteuert wird, benötigt abhängig von seiner Bandbreite eine gewisse Zeit, bis er abgeklungen ist und wieder neu angesteuert werden kann. Diese Zeit ist i. a. länger als das Zeitfenster des Empfängers, so daß zwischen den zur Messung zur Verfügung stehenden Zeitintervallen Totzeiten auftreten, während denen kein Meßsignal vorliegt. Bei einer nichtstationären Messung, beispielsweise wenn als Meßgut ein Blatt Papier an der Sender-Empfänger-Anordnung vorbeibewegt wird, ist das örtliche Auflösungsvermögen der bekannten Vorrichtung von den Totzeiten bzw. der Impulswiederholfrequenz abhängig. Mit steigender Geschwindigkeit des Meßgutes können daher kleine örtliche Abweichungen des Flächengewichts nur noch begrenzt aufgelöst werden.

Der Erfindung liegt daher die Aufgabe zugrunde, eine Vorrichtung zur Bestimmung des Flächengewichts von blattförmigem Material vorzuschlagen, mit der Störungen des Meßsignals aufgrund der Überlagerung von Schallwellen vermieden werden und mit der insbesondere bei schnellbewegtem Meßgut relativ zur Sender-Empfänger-Anordnung eine hohe örtliche Auflösung erreicht werden kann.

Erfindungsgemäß wird die Aufgabe durch die im kennzeichnenden Teil des Hauptanspruchs angegebenen Merkmale gelöst.

Der Grundgedanke der Erfindung besteht darin, alle an der Messung beteiligten Elemente wie Sender, Empfänger und Meßgut derart anzuordnen, daß die an diesen Elementen reflektierten Schallanteile aus dem Strahlengang zwischen Sender und Empfänger ausgeblendet werden und gleichzeitig durch geeignete Mittel verhindert wird, daß die ausgeblendeten Schallanteile wieder in den ursprünglichen Strahlengang zurückkehren und auf Sender und/oder Empfänger treffen. Dieser Grundgedanke kann beispielsweise dadurch realisiert werden, daß das Meßgut nicht parallel, sondern unter einem Winkel zu Sender und Empfänger angeordnet ist und der am Meßgut reflektierte Schall durch ein schallabsorbierendes Material vernichtet wird.

Aus der EP-A 00 98 115 ist zwar eine Vorrichtung bekannt, bei der Sender und Empfänger schräg zum Meßgut angeordnet sind, diese Vorrichtung ist aber nicht geeignet, die der Erfindung zugrundeliegenden Probleme zu lösen.

Da durch die erfindungsgemäße Anordnung im Strahlengang zwischen Sender und Empfänger zu keinem Zeitpunkt störende Überlagerungen von Schallwellen auftreten können, ist der Zeitpunkt der Messung sowie ein eventuelles Zeitfenster des Empfängers oder die Impulswiederholfrequenz ohne Auswirkung auf das Meßsignal. Die vorgenannten Größen können daher beliebig an die jeweiligen Verhältnisse angepaßt werden.

Insbesondere ist es auch möglich, mit Dauerschall zu arbeiten, d. h. mit einer kontinuierlich abgestrahlten Senderschallwelle. Die Anwendung von Dauerschall bietet den Vorteil, daß keine Totzeiten auftreten und somit eine kontinuierliche Messung möglich ist. Dadurch wird eine hohe örtliche Auflösung in Bewegungsrichtung des Meßgutes erreicht, auch wenn sich das Meßgut schnell an der Sender-Empfänger-Anordnung vorbeibewegt.

Ein weiterer Vorteil der erfindungsgemäßen Anordnung der Elemente besteht darin, daß die Lage des Meßguts bezüglich Sender und Empfänger keinen Einfluß auf das Meßsignal ausübt. Bei den bekannten Vorrichtungen mit parallel zu Sender und Empfänger angeordnetem Meßgut treten i. a. Transmissionsänderungen auf, wenn das Meßgut in Richtung der Verbindungsachse Sender - Empfänger bewegt wird. Dieser unerwünschte Effekt kommt durch Interferenzerscheinungen der reflektierten Schallwellen zustande, wobei je nach Phasenlage der interferierenden Schallwellen die Transmission schwankt. Mit der vorgeschlagenen Lösung wird dieser Nachteil beseitigt, was sich insbesondere dann positiv auswirkt, wenn die Lage des Meßgutes bezüglich Sender und Empfänger nicht konstant gehalten werden kann, beispielsweise wenn das Meßgut durch eine schnelle Bewegung zum Flattern neigt.

Weitere Vorteile sowie Weiterbildungen der Erfindung ergeben sich aus den Unteransprüchen sowie aus der nachfolgenden Beschreibung von Ausführungsbeispielen der Erfindung anhand der beigefügten Zeichnungen.

Darin zeigen:
- Fig. 1: eine schematische Darstellung einer Vorrichtung zur Flächengewichtsbestimmung von blattförmigem Material,
- Fig. 2: die Anordnung der erfindungsgemäßen Vorrichtung in einem Gehäuse,
- Fig. 3: eine weitere Ausführungsform mit zusätzlicher Einengung des Bündelquerschnitts,
- Fig. 4: eine Platte zur Einengung des Strahlbündels in Schnittdarstellung und
- Fig. 5: eine schematische Darstellung einer Vorrichtung in Reflexionsanordnung.

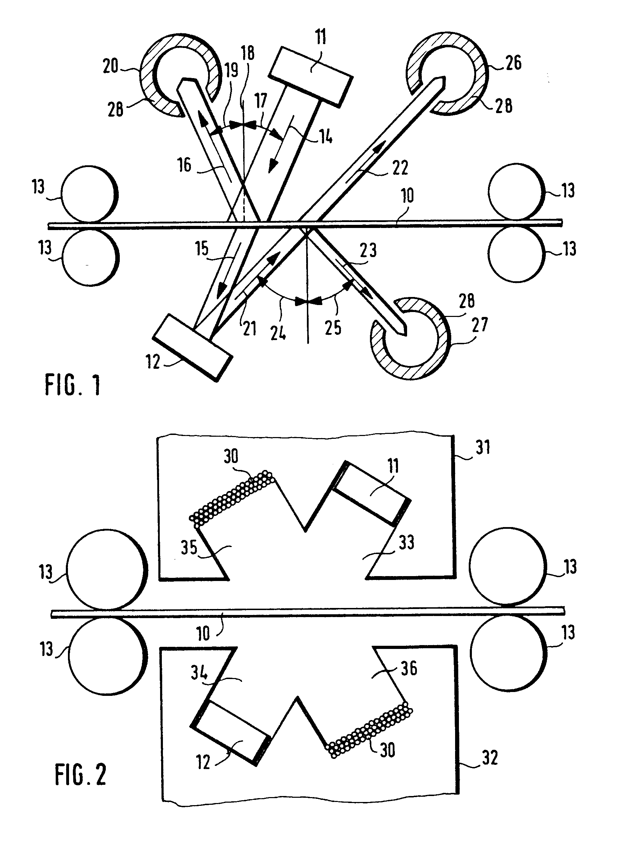

Die Fig. 1 zeigt in schematisierter Darstellung ein Beispiel für die erfindungsgemäße Vorrichtung. Das Meßgut 10, beispielsweise ein Blatt Papier, wird mit Hilfe von Führungselementen 13, wie den in der Fig. angedeuteten Transportrollen, zwischen Sender 11 und Empfänger 12 hindurchbewegt. Sender 11 und Empfänger 12 sind relativ zu den Führungselementen 13 derart angeordnet, daß der vom Sender in Richtung des Pfeils 14 abgestrahlte Schall unter einem von Null verschiedenen Winkel 17, bezogen auf das Lot 18 im Auftreffpunkt des Senderschalls 14, auf das Meßgut 10 trifft.

Die Richtung des reflektierten Schallanteils, angedeutet durch den Pfeil 16, ist durch die Reflexionsbedingung (Einfallswinkel 17 gleich Ausfallswinkel 19) festgelegt. Da der Einfallswinkel 17 erfindungsgemäß von Null, d. h. von senkrechter Beschallung, abweicht, wird die am Meßgut reflektierte Schallwelle 16 am Sender 11 vorbeigelenkt. Geeignete Maßnahmen, beispielsweise eine schallabsorbierend wirkende Vorrichtung 20 (Schallfalle) sorgen dafür, daß die Schallwelle 16 nicht mehr in den ursprünglichen Strahlengang zwischen Sender und Empfänger zurückkehren kann. Als Schallfalle ist z. B. ein mit einer Öffnung versehener Hohlkörper anwendbar, der innen mit schalldämpfendem Material 28 ausgekleidet ist. Schall, der durch die Öffnung eintritt, wird größtenteils durch das schalldämpfende Material 28 absorbiert; nichtabsorbierter Schall wird reflektiert, verbleibt aber im Innern des Hohlraums und wir schließlich ebenfalls absorbiert. In der Praxis hat sich herausgestellt, daß anstelle des "schallschluckenden Hohlraums" bereits die bloße Verwendung eines schallabsorbierenden Materials, beispielsweise eines Stücks Schaumstoffs, gute Ergebnisse liefert.

Anstelle von schallabsorbierenden Mitteln 20 ist es auch möglich, den am Meßgut reflektierten Schall 16 durch geeignete Anordnung der Elemente oder durch zusätzliche Einrichtungen, wie z. B. Schallspiegel, so zu leiten, daß der Schall 16 am Sender vorbei in den freien Raum reflektiert wird und damit verlorengeht. Beispielsweise wirkt ein Loch in einer Gehäusewand in gleicher Weise wie ein ideales Absorptionsmaterial an gleicher Stelle.

Der durch das Meßgut 10 transmittierte Schallanteil 15 trifft auf den Empfänger 12, wird von diesem teilweise absorbiert und in ein elektrisches Meßsignal umgewandelt. Der nichtabsorbierte, reflektierte Schallanteil ist in Fig. 1 durch den Pfeil 21 angedeutet. Im allgemeinen ist der Empfänger 12 gegenüber der Richtung der einfallenden Schallwelle 15 derart geneigt, daß die am Empfänger reflektierte Schallwelle 21 eine von der einfallenden Welle 15 unterschiedliche Richtung aufweist. Dadurch wird verhindert, daß es zu Überlagerungen zwischen der vom Sender abgestrahlten Welle 14 bzw. 15 und der reflektierten Welle 21 kommt, die zu stehenden Wellen führen können. Ebenso wird verhindert, daß die Schallwelle 21 auf den Sender zurückkommt, von diesem reflektiert wird und nochmals auf den Empfänger trifft. In beiden Fällen kann das Meßsignal und damit die Flächengewichtsbestimmung verfälscht werden.

Die aus dem Strahlengang Sender - Empfänger wegreflektierte Schallwelle 21 wird entweder direkt, d. h. vor Auftreffen auf das Meßgut, durch eine Schallfalle absorbiert oder trifft, wie in Fig. 1 dargestellt, nochmals auf das Meßgut. In Analogie zu den oben gemachten Ausführungen wird ein Teil 22 der Schallwelle 21 durch das Meßgut transmittiert und der restliche Anteil 23 reflektiert, wobei wiederum das Reflexionsgesetz (Einfallswinkel 24 gleich Ausfallswinkel 25) die Richtung der reflektierten Schallwelle 23 festlegt. Schallfallen 26, 27 absorbieren die Schallwellen 22, 23, die für die Messung ohne Bedeutung sind und nur zu Störsignalen führen könnten.

Durch die Schrägstellung des Empfängers bezüglich der einfallenden Schallwelle 15 sind die Winkel 17 und 24 unterschiedlich, wobei der Winkel 24 je nach Neigung des Empfängers größer oder kleiner als der Winkel 17 sein kann.

In Fällen, wo das Meßgut nur kleine Transmissionsgrade aufweist (z. B. Papier, Transmission im Bereich weniger %, können Sender und Empfänger auch parallel angeordnet werden, ohne das Meßergebnis zu verfälschen. Durch die geringe Transmission ist der Einfluß der am Empfänger reflektierten Schallwelle 21, die bei paralleler Sender-Empfänger-Anordnung auf den Sender zurückkommt, von diesem reflektiert wird und ein zweites Mal auf den Empfänger gelangt, völlig vernachlässigbar (kleiner als 10⁻⁵ bei einer angenommenen Transmission von 2 %).

Die am Empfänger reflektierte Schallwelle 21 wird bei nichtgeneigter Sender-Empfänger-Anordnung zwar in den ursprünglichen Strahlengang zurückreflektiert, sie bleibt auf das Meßsignal jedoch ohne Einfluß, da die Schallwelle 23 durch die Reflexion am Meßgut so abgelenkt wird, daß die Welle 23 nicht mehr auf den Empfänger gelangen kann. In diesem Fall sind die Winkel 17 und 24 gleich groß. Die Überlagerung der einfallenden Welle 15 und der am Empfänger reflektierten Welle 21 führt zwischen Empfänger und Meßgut zu einer stehenden Welle. Einfallende und reflektierte Welle überlagern sich dabei ohne gegenseitige Beeinflussung, solange keine Brechung der einfallenden Welle 15 an den durch die stehende Welle erzeugten Dichteschwankungen der Luft auftritt. Bei den verwendeten Schalldrücken ist man aber von diesem Fall einer Selbstbrechung noch weit entfernt.

Die Größe der Winkel 17 und 24 ist in weiten Grenzen frei wählbar und kann an die jeweiligen Bedingungen, beispielsweise die geometrischen Abmessungen eines Gehäuses, angepaßt werden. Zu beachten ist, daß sich bei vorgegebenem Durchmesser des vom Sender abgestrahlten Schallbündels die beschallte Fläche auf dem Meßgut bei schräger Beschallung gegenüber senkrechter Beschallung vergrößert. Für eine große örtliche Auflösung wird man daher in der Regel den Winkel 17 klein wählen. Mit einem Winkel 17 von 22,5° wurden in der Praxis gute Meßergebnisse erreicht.

In Fig. 2 ist gezeigt, wie das in Fig. 1 schematisch dargestellte Verfahren zur Flächengewichtsbestimmung in der Praxis für Messungen an Papier angewendet werden kann. Da die Transmission für Papier i.a. nur sehr kleine Werte annimmt, könnten aus den o.g. Gründen Sender und Empfänger parallel zueinander aufgestellt werden. Im allgemeinen sind Sender 11 und Empfänger 12 zu ihrem Schutz und zur Befestigung in Gehäusen 31, 32 zu beiden Seiten des Meßguts angeordnet. Die beiden Gehäuse können gemeinsam auf einer Grundplatte befestigt sein. Die Form bzw. Ausstattung der Gehäuse muß dabei so sein, daß die am Meßgut reflektierten Schallwellen durch mehrmalige Reflexionen am Gehäuse nicht wieder in den ursprünglichen Strahlengang und insbesondere nicht auf den Empfänger gelangen, um das Meßsignal nicht zu verfälschen. Dies kann zum Beispiel durch Einbau von absorbierenden Schallfallen an diejenigen Stellen erfolgen, in welche die Schallwellen reflektiert werden. In Fig. 2 sind in Richtung der reflektierten Sender- und Empfänger-Schallanteile zwei Kanäle 35, 36 vorgesehen, in welche der Schall eintreten kann. Die Querschnitte der Kanäle 35, 36 sind vorzugsweise etwas größer als die Querschnitte der Kanäle 33,34, die direkt vom Sender zum Empfänger führen. Dadurch kann sichergestellt werden, daß der gesamte vom Sender kegelförmig abgestrahlte und vom Meßgut reflektierte Schall in den jeweiligen Kanal eintritt. Am Ende der Kanäle 35, 36 befindet sich ein schallabsorbierendes Material 30.

Als besonders wirkungsvolles Material hat sich in der Praxis ein offenporiger Polyurethanschaumstoff erwiesen. Die Hohlräume dieses Schaumstoffs liegen im Bereich von 1 mm und sind damit vergleichbar mit der Wellenlänge des verwendeten Ultraschalls, die bei 200 kHz etwa 1,5 mm beträgt. In speziellen Fällen kann der Abschluß der Schallfalle auch mit pyramidenförmigen Schallabsorbern gebildet werden, wie sie beispielsweise von reflektionsarmen (schalltoten) Räumen bekannt sind.

Die schallführenden Kanäle 33 - 36 können aus dem Gehäusematerial selbst bestehen (beispielsweise gefräst oder gebohrt in Aluminium), vorzugsweise wird man aber auch die Kanäle für die Schallführung mit einem schalldämpfenden Material versehen. Dadurch wird erreicht, daß nur die direkte, in einem engen Schallkegel abgestrahlte Schallwelle auf den Empfänger gelangt, während Reflexionen an den Wänden der schallführenden Kanäle unterdrückt werden.

Die Führungselemente 13 sind außerhalb des Sender- und Empfänger-Gehäuses angeordnet, so daß die Gehäuse 31, 32 nahe an das Meßgut heranreichen und damit nur einen schmalen Spalt bilden. Dadurch wird erreicht, daß einerseits Störungen (z. B. allgemeiner Lärm) von außerhalb der Meßvorrichtung keinen oder nur geringen Einfluß auf die Messung ausüben und andererseits der Schall der Meßvorrichtung nicht nach außen dringen und seinerseits Störungen verursachen kann. Außerdem kann die Verschmutzung der Vorrichtung durch den schmalen Spalt herabgesetzt werden.

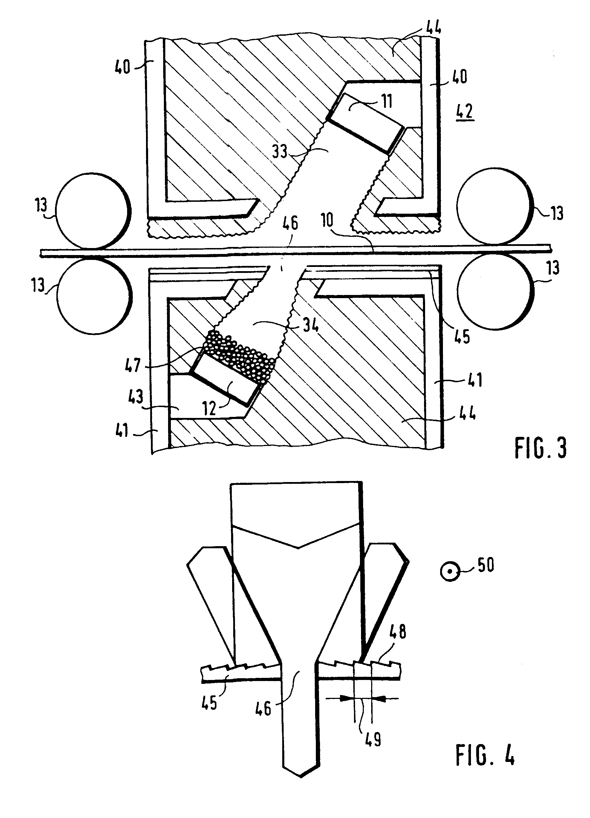

In Fig. 3 ist eine weitere Ausführungsform dargestellt, wie sie beispielsweise zur Flächengewichtsbestimmung von Banknoten benutzt werden kann. Die prinzipielle Anordnung von Sender, Empfänger und Meßgut ist bereits aus den beiden vorangegangenen Fig. bekannt und wird im einzelnen nicht mehr beschrieben. Gleiche Elemente sind in den Fig. mit gleichen Positionsangaben versehen. Das Sendergehäuse besteht aus einer geeignet geformten, stabilen Gehäusewand 40, beispielsweise aus Aluminium. An einem mit der Gehäusewand 40 verbundenen Träger 42 ist der Schallsender 11 befestigt. Die Gehäusewand 40 weist an geeigneter Stelle eine Öffnung für den Austritt des Senderschalls auf. Das Innere des Sendergehäuses ist teilweise oder, wie in Fig. 3 dargestellt, ganz mit schallabsorbierendem Material ausgekleidet. Der vom Sender wegführende Schallkanal 33 wird durch ein schallabsorbierendes Material 44 gebildet bzw. ist mit diesem Material ausgekleidet.

In der Praxis hat sich herausgestellt, daß für den vom Meßgut reflektierten Schallanteil kein separater Kanal (wie durch 35 in Fig. 2 dargestellt) vorgesehen werden muß, wenn, wie in der Fig. 3 gezeigt, das Sendergehäuse auch an den äußeren Stellen, an denen reflektierte Schallanteile auftreffen können, mit schallabsorbierendem Material genügender Dicke versehen ist.

Prinzipiell ist für die Empfängerseite dieselbe Ausgestaltung des Gehäuses wie für die Senderseite vorgesehen, also mit Gehäusewand 41, Träger 43 für Empfänger-Schallwandler und schalldämpfendem Material 44. Zusätzlich ist in Fig. 3 eine Platte 45 gezeigt, die eine Öffnung 46 aufweist, die kleiner als der Durchmesser des vom Sender abgestrahlten Schallkegels ist. Zweck dieser Platte 45 ist es, den Querschnitt des vom Sender kommenden Schallbündels, der für die räumliche Auflösung verantwortlich ist, in der Nähe des Meßgutes zu verkleinern. Dies ist insbesondere dann notwendig, wenn der Sender bzw. Empfänger aufgrund ihrer Bauart nicht beliebig verkleinert werden können. Bekanntlich sind der Durchmesser und die Wellenlänge von Ultraschallwandlern in gewissen Grenzen zueinander proportional. Durch Anwendung der Platte 45 kann also das örtliche Auflösungsvermögen der Vorrichtung unabhängig vom Durchmesser der Schallwandler durch Verkleinerung der Plattenöffnung 46 erhöht werden. Im gezeigten Beispiel besteht die Platte 45 z. B. aus Aluminium. Für diesen Fall sind Mittel vorzusehen, durch welche der auf die Platte auftreffende Schall absorbiert oder aus dem Strahlengang zwischen Sender und Empfänger ausgeblendet wird. Neben einer Beschichtung der Platte mit schallabsorbierendem Material kann die Platte so ausgebildet sein, daß auftreffende Schallanteile aus dem Meßkanal wegreflektiert werden. Die Funktion einer derartigen Platte wird im Zusammenhang mit Fig. 4 noch ausführlich beschrieben.

Weiterhin ist auf der dem Sender zugewandten Seite des Empfänger-Schallwandlers eine schalldämpfende Schicht 47 angebracht. Diese dient dazu, den vom Empfänger reflektierten Schallanteil zu vermindern. Dadurch wird einerseits erreicht, daß möglichst wenig Schallenergie an die dem Gehäuseinneren zugewandten Seite der Platte 45 vom Empfänger zurückreflektiert wird, was insbesondere dann von Vorteil ist, wenn die Öffnung der Platte 45 kleiner als der Durchmesser der Schallwandler ist. Andererseits erreicht man eine bessere Anpassung der Schallwiderstände von Luft und dem des Schallwandlers.

Ein weiterer Vorteil der auf dem Empfänger-Schallwandler aufgebrachten Schicht 47 tritt dann in Erscheinung, wenn ein gut transmittierendes Meßgut untersucht wird. Bei hohen Transmissionswerten des Meßguts treten nämlich bei paralleler Anordnung der aktiven Flächen von Sender und Empfänger (wie z. B. in den Fig. 2 und 3) Überlagerungen der vom Empfänger reflektierten Schallwelle mit der vom Sender abgestrahlten Schallwelle auf. Bei geeignetem Verhältnis von Frequenz und Sender-Empfänger-Abstand, d. h. bei passender Phasenlage der hin- und zurücklaufenden Wellen können sich zwischen Sender und Empfänger stehende Wellen ausbilden. Je nachdem, ob sich das Meßgut nun in einem Schwingungsbauch oder einem Schwingungsknoten der stehenden Welle befindet, ändert sich die Transmission des Meßguts zwischen einem Maximal- und einem Minimalwert. Die Differenz der beiden Extremwerte hängt entscheidend von den Reflexionsfaktoren der beteiligten Elemente (Sender, Empfänger und Meßgut) ab. Je kleiner die Reflexionsfaktoren sind, desto weniger unterscheiden sich der Maximal- und der Minimalwert. Um also gegenüber Verschiebungen des Meßguts auf der Verbindungslinie Sender-Empfänger unempfindlich zu werden, ist es sinnvoll, die Reflexionswerte der beteiligten Elemente möglichst klein zu wählen. Da der Reflexionsfaktor des Meßgutes vorgegeben ist, können nur die Reflexionsfaktoren von Sender und Empfänger variiert werden. Auf der Senderseite wird man allerdings kein zusätzliches, schallabsorbierendes Material auf den Schallwandler aufbringen, da hierdurch die vom Sender abgestrahlte Schallenergie und dadurch das Signal-Rausch-Verhältnis verringert würde. Auf der Empfängerseite dagegen ist die Anbringung von schalldämpfendem Material ohne Einfluß auf das Signal-Rausch-Verhältnis, da sowohl die Meßschallwelle als auch Störgeräusche in gleichem Maß abgeschwächt werden und das Signal-Rausch-Verhältnis nicht beeinflußt wird.

Die Verwendung von schallabsorbierendem Material 47 auf der aktiven Fläche des Empfängers hat den weiteren Vorteil, daß bei einem 100 %-Abgleich der Sender-Empfänger-Anordnung, d. h. ohne dazwischenliegendes Meßgut, Interferenzen zwischen Sender und Empfänger vermieden bzw. deren Auswirkungen verringert werden. Ohne das schallabsorbierende Material 47 könnten sich stehende Wellen zwischen Sender und Empfänger ausbilden, wenn deren Abstand ein Vielfaches der halben Schallwellenlänge beträgt. Stehende Wellen stören aber das Meßsignal und sind daher zu vermeiden.

Als schalldämpfendes Material 47 für die Anbringung auf dem Schallempfänger hat sich wiederum eine dünne Schicht des bereits oben erwähnten offenporigen Polyurethan-Schaumstoffs bewährt.

In der in Fig. 3 gezeigten Vorrichtung wird die Reflexion der vom Empfänger reflektierten Schallwelle an der Platte 45 weiterhin dadurch vermieden, daß die Platte auf der dem Gehäuseinneren zugewandten Seite mit schalldämpfendem Material beschichtet ist. Bei Verwendung einer Platte 45 mit im Vergleich zum Schallwandlerdurchmesser kleinen Öffnung kann der Schallkanal zwischen der Öffnung 46 in der Platte 45 und dem Empfänger, wie in Fig. 3 gezeigt, vorzugsweise kegelförmig ausgebildet sein.

Anstelle der vollständigen Ausfüllung des durch die Gehäusewand gebildeten Hohlraums mit schallabsorbierendem Material auf der Empfängerseite, wie in der Fig. 3 gezeigt, ist es auch möglich, nur die notwendigen Bereiche, also insbesondere den Schallkanal 34 und die Unterseite der Platte 45 im Bereich der Öffnung 46 mit schallabsorbierendem Material 30 zu versehen.

In Fig. 4 ist die Platte 45 im Schnitt dargestellt. Der Schnitt liegt in einer zur Zeichenebene der Fig. 3 senkrechten Ebene, d. h. die Ansicht nach Fig. 4 erhält man bei Betrachtung der Platte in Bewegungsrichtung 50 des Meßgutes. Der einfallende Senderschall besitzt einen Durchmesser, der im wesentlichen durch den Schallwandler vorgegeben ist. Zur Einengung des Bündelquerschnitts weist die Platte 45 eine Öffnung 46 auf. Hinter der Platte besitzt das Schallbündel einen kleineren Durchmesser, womit ein höheres örtliches Auflösungsvermögen erreicht wird.

Die Plattenoberseite, die dem einfallenden Schall zugewandt ist, ist so ausgebildet, daß der nicht durch die Öffnung 46 durchgehende Schall aus der durch das Lot 18 und dem Senderschall 14 gebildeten Meßebene (die in Fig. 4 senkrecht zur Zeichenebene steht) wegreflektiert wird. Im einfachsten Fall erreicht man dies durch eine geneigte Oberfläche der Platte. Nachteilig bei nur einer geneigten Fläche (z. B. kegelförmig oder ähnlich einem Satteldach) ist, daß die Platte in der Nähe der Öffnung relativ dick und in den Randbereichen relativ dünn ausgebildet sein würde, wenn man einen für die Praxis annehmbaren, großen Reflexionswinkel fordert. Vorteilhafter ist eine Aufteilung in mehrere Bereiche, wie dies in Fig. 4 dargestellt ist. An den einzelnen kleinen schräggestellten Bereichen 48 wird der Schall unter Reflexionsbedingungen aus der Einfallsrichtung wegreflektiert.

Wenn die Wellenlänge der Schallwelle etwa derselben Größe wie der Abstand 49 benachbarter Teilbereiche 48 beträgt, treten zusätzlich Beugungserscheinungen auf.

Da man einzelne Punkte der Platte 45 als selbständige Schallsender auffassen kann, gibt es aufgrund der Phasenlage der punktförmigen Sender untereinander ausgezeichnete Richtungen, sogenannte Beugungsanordnungen, in welche die Schallwellen konstruktiv interferieren. Wenn die Richtung einer Beugungswelle mit der Reflexionsrichtung zusammenfällt, erhöht sich die Effizienz der aus dem Einfallsstrahl weggebeugten Schallenergie. Erreichbar ist dieser Fall hoher Effizienz durch Anpassung der Abstände 49 an die Schrägstellung der Bereiche 48 bzw. umgekehrt.

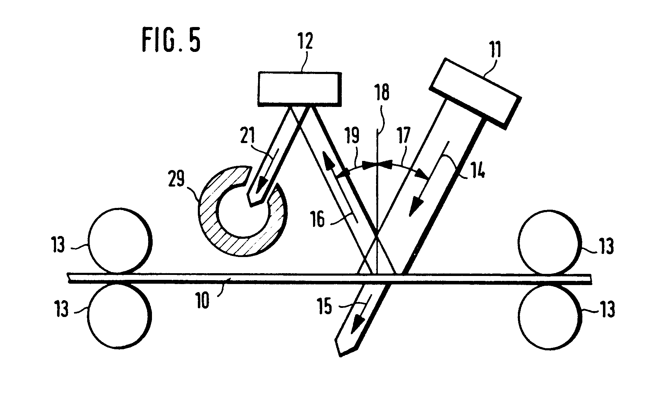

Die erfindungsgemäße Schrägstellung der Elemente in Kombination mit zusätzlichen Mitteln, die verhindern, daß reflektierte Schallanteile in den Strahlengang zwischen Sender und Empfänger zurückkehren können, kann auch bei einer Reflexionsmessung vorteilhaft angewendet werden. Eine schematische Darstellung einer derartigen Vorrichtung zeigt Fig. 5. Gleiche Elemente sind mit gleichen Positionsangaben wie in den vorangegangenen Fig. versehen.

Bei einer Reflexionsmessung trifft der vom Sender 11 ausgehende Schall 14 unter einem Winkel 17 auf das Meßgut 10. Ein Teil 15 des Senderschalls wird vom Meßgut transmittiert; der verbleibende und vom Meßgut nicht absorbierte Anteil wird aufgrund des Reflexionsgesetzes unter einem Winkel 19 in die Richtung 16 reflektiert und trifft auf den Empfänger 12. Die aktive Fläche des Empfängers 12 ist gegenüber der Richtung der ankommenden Schallwelle 16 derart geneigt, daß die Richtung der am Empfänger reflektierten Schallwelle 21 von der Richtung der ankommenden Schallwelle 16 abweicht. Eine Schallfalle 29 absorbiert den am Empfänger reflektierten Schallanteil.

Da der am Empfänger reflektierte Schallanteil 21 nicht mehr in den zur Messung dienenden Strahlengang, der durch die Richtungen der Schallwellen 14 und 16 gegeben ist, zurückkehren kann, treten keine störenden Interferenzen in diesem Strahlengang auf. So werden beispielsweise stehende Wellen zwischen Sender, Empfänger und dem teilweise als Reflektor wirkenden Meßgut ebenso vermieden wie Vielfachreflexionen, die sich dem eigentlichen Meßsignal überlagern und dieses verfälschen können.

## Patentansprüche

1. Vorrichtung zur dynamischen Bestimmung des lokalen Flächengewichts von blattförmigem Material (10), beispielsweise Papier, bei der das blattförmige Material mit Schallwellen aus einem Schallsender (11) beaufschlagt wird, der durch das blattförmige Material transmittierte und/oder reflektierte Schallanteil mit Hilfe eines Empfängers (12) gemessen und aus dem Meßsignal das Flächengewicht bestimmt wird, dadurch **gekennzeichnet,** daß Sender (11), Meßgut (10) und gegebenenfalls Empfänger (12) derart schräg zueinander angeordnet sind, daß die an diesen Elementen reflektierten Schallanteile aus dem Strahlengang zwischen Sender (11) und Empfänger (12) ausgeblendet werden und daß zusätzlich Mittel (20, 26, 27, 29) vorgesehen sind, die verhindern, daß die ausgeblendeten Schallanteile wieder in den Strahlengang zwischen Sender (11) und Empfänger (12) zurückkehren können.

2. Vorrichtung nach Anspruch 1, dadurch **gekennzeichnet,** daß die Mittel (20, 26, 27, 29) schallabsorbierend wirkende Mittel sind.

3. Vorrichtung nach Anspruch 2, dadurch **gekennzeichnet,** daß die schallabsorbierend wirkenden Mittel (20, 26, 27, 29) aus schalldämpfendem Schaumstoff bestehen.

4. Vorrichtung nach Anspruch 1, dadurch **gekennzeichnet,** daß die Mittel (20) Schallreflektoren sind, die die ausgeblendeten Schallanteile in den freien Raum leiten.

5. Vorrichtung nach Anspruch 1, dadurch **gekennzeichnet,** daß der Sender (11) ein kontinuierlich betriebener Ultraschallsender ist.

6. Vorrichtung nach einem der Ansprüche 1 - 5, dadurch **gekennzeichnet,** daß Sender (11) und Empfänger (12) einander gegenüberliegend angeordnet sind und daß eine gedachte Verbindungslinie zwischen Sender (11) und Empfänger (12) mit dem Lot (18) auf das Meßgut (10) etwa einen Winkel von 10° bis 60°, vorzugsweise 22,5°, einschließt.

7. Vorrichtung nach Anspruch 6, dadurch **gekennzeichnet,** daß die aktive Fläche des Empfängers (12) bezüglich der gedachten Verbindungslinie zwischen Sender (11) und Empfänger (12) senkrecht angeordnet und mit einem schallabsorbierenden Material (47) versehen ist.

8. Vorrichtung nach Anspruch 6, dadurch **gekennzeichnet,** daß die aktive Fläche des Empfängers (12) bezüglich der gedachten Verbindungslinie zwischen Sender (11) und Empfänger (12) geneigt ist.

## Claims

1. An apparatus for dynamically determining the local weight per unit area of sheet material (10), for example paper, wherein the sheet material is exposed to sound waves from a sound transmitter (11), the sound fraction transmitted and/or reflected by the sheet material is measured by aid of a receiver (12) and the weight per unit area is determined from the measuring signal, characterized in that the transmitter (11), the material under test (10) and possibly the receiver (12) are arranged on a slant relative to one other in such a way that the sound fractions reflected on these elements are faded out of the path of rays between the transmitter (11) and the receiver (12), and means (20, 26, 27, 29) are additionally provided to prevent the faded out sound fractions from returning to the path of rays between the transmitter (11) and the receiver (12).

2. The apparatus of claim 1, characterized in that the means (20, 26, 27, 29) are means having a sound-absorbing effect.

3. The apparatus of claim 2, characterized in that the means (20, 26, 27, 29) having a sound-absorbing effect are made of sound-absorbing foamed material.

4. The apparatus of claim 1, characterized in that the means (20) are sound reflectors which conduct the faded out sound fractions into the free space.

5. The apparatus of claim 1, characterized in that the transmitter (11) is a continuously operated supersonic transmitter.

6. The apparatus of claim 1, characterized in that the transmitter (11) and receiver (12) are arranged opposite each another, and an imaginary connecting line between the transmitter (11) and receiver (12) forms an angle approximately between 10° and 60°, preferably 22.5°, with the vertical (18) onto the material under test (10).

7. The apparatus of claim 6, characterized in that the active surface of the receiver (12) is arranged at right angles to the imaginary connecting line between the transmitter (11) and receiver (12) and is provided with a sound-absorbing material (47).

8. The apparatus of claim 6, characterized in that the active surface of the receiver (12) is on a slant with respect to the imaginary connecting line between the transmitter (11) and receiver (12).

## Revendications

1. Dispositif pour la détermination dynamique de la masse surfacique locale d'un matériau en feuille (10), par exemple de papier, dans lequel le matériau en feuille est soumis à des ondes sonores à partir d'un émetteur acoustique (11), la partie de l'émission sonore transmise et/ou réfléchie par Le matériau en feuille est mesurée à l'aide d'un récepteur (12) et la masse surfacique est déterminée à partir du signal mesuré, caractérisé en ce que l'émetteur (11), le matériau à mesurer (10) et, le cas échéant, le récepteur (12) sont disposés en biais les uns par rapport aux autres, de telle façon que la partie de l'émission sonore réfléchie sur ces éléments soit extraite sur le parcours d'émission entre l'émetteur (11) et le récepteur (12) et en ce que sont prévus des moyens supplémentaires (20, 26, 27, 29) qui empêchent que la partie d'émission sonore extraite puisse revenir sur le parcours d'émission entre l'émetteur (11) et le récepteur (12).

2. Dispositif selon la revendication 1, caractérisé en ce que les moyens (20, 26, 27, 29) sont constitués par des moyens absorbant les sons.

3. Dispositif selon la revendication 2, caractérisé en ce que les moyens absorbant les sons (20, 26, 27, 29) comportent un matériau mousse amortissant les sons.

4. Dispositif selon la revendication 1, caractérisé en ce que les moyens (20) sont des réflecteurs sonores qui conduisent à l'air libre la partie d'émission sonore extraite.

5. Dispositif selon la revendication 1, caractérisé en ce que l'émetteur (11) est un émetteur d'ultrasons actionné en continu.

6. Dispositif selon l'une des revendications 1 à 5, caractérisé en ce que l'émetteur (11) et le récepteur (12) sont disposés opposés l'un à l'autre et en ce qu'une ligne imaginaire de liaison entre l'émetteur (11) et le récepteur (12) fait avec la perpendiculaire (18) au matériau à mesurer (10) un angle d'environ 10 à 60°, de préférence 22,5°.

7. Dispositif selon la revendication 6, caractérisé en ce que la surface active du récepteur (12) est disposée perpendiculairement à la ligne imaginaire de liaison entre l'émetteur (11) et le récepteur (12) et est munie d'un matériau (47) absorbant les sons.

8. Dispositif selon la revendication 6, caractérisé en ce que la surface active de l'émetteur (12) est inclinée par rapport à la ligne imaginaire de liaison entre l'émetteur (11) et le récepteur (12).
